# EUROPEAN PATENT APPLICATION

(11) **EP 2 259 064 A2**
(43) Date of publication of application: **08.12.2010**
(21) Application number: 10163517.5
(22) Date of filing: 21.05.2010
(51) Int. Cl.: G01N 33/569, C07K 5/06

(54) **Peptide compounds for detecting or inhibiting SARS coronavirus and application thereof**

(30) Priority: 27.05.2009 KR 20090046309
(71) Applicant: Electronics and Telecommunications Research Institute, Daejeon-city 305-350 (KR)
(72) Inventor: Lee, Soo-Hyung, 305-721 Daejeon (KR); Hong, Hyo-Bong, 305-755 Daejeon (KR); Kim, Tae-Wan, 302-737 Daejeon (KR); Chung, Myung-Ae, 305-720 Daejeon (KR); Sohn, Sung-Won, 305-350 Daejeon (KR); Yoo, Seoung-Min, 301-790 Daejeon (KR)
(74) Representative: Betten & Resch

(57) **Abstract**

Disclosed herein are peptide compounds and the application thereof to the detection and inhibition of SARS coronavirus. Composed of dipeptides, the compounds for detecting and inhibiting SARS coronavirus can be readily synthesized and produced at low cost. In addition, they can be stored safely for a long period of time. The dipeptide compounds are useful as inhibitors of SARS coronavirus as well as acting as excellent capturing materials of SARS coronavirus.

## Description

### RELATED APPLICATIONS

The present application claims priority to Korean Patent Application Serial Number 10-2009-0046309, filed on May 27,2009, the entirety of which is hereby incorporated by reference.

### BACKGROUND OF THE INVENTION

### 1. Field of the Invention

The present invention relates to peptide compounds for the detection or inhibition of SARS coronavirus and to the application thereof.

### 2. Description of the Related Art

The epidemic of Severe Acute Respiratory Syndrome (SARS) appears to have started in Guangdong Province, China in November 2002 and spread therefrom to rapidly infect individuals in some countries around the world including Hongkong, Singapore, Vietnam, Canada, and the U.S.A., with more than 8,000 known infected cases and more than 700 deaths worldwide. When infected with SARS coronavirus, patients suffer from symptoms including fever, cough, dyspnea, atypical pneumonia, etc. SARS coronavirus is known as a coronavirus variant different from typical human coronaviruses. Now, the spread of SARS has been fully contained thanks to the efforts of the WHO, with no infected human case seen since then, but there is still the possibility that it may potentially return into the human population in the future. As SARS coronavirus propagates from one person to another via respiratory infection, such as droplet infection, the epidemic of SARS, if it were to restart, would be apt to lead to a pandemic, causing serious damage all over the world.

For the detection or diagnosis of SARS, ELISA using the blood is usually used to examine the presence of antigens or antibodies. SARS Diagnosis or detection by ELISA needs at least 7 days after the appearance of symptoms of infection. For this reason, preference is given to PCR for detecting viral genes. Further, ELISA must be supported by techniques for producing antigens or antibodies and are difficult to apply to tools such as bio-sensors because it uses bio-materials.

On the other hand, the use of viral genes makes PCR highly sensitive as pertains to the diagnosis of SARS viral genes. PCR is expensive to operate. In addition, it is difficult to immediately diagnose SARS with PCR because it requires three or more hours for analysis.

### SUMMARY OF THE INVENTION

Accordingly, the present invention has been made keeping in mind the above problems occurring in the prior art, and an object of the present invention is to provide a capturing compound which can be used in lieu of antibodies in the detection of SARS coronavirus, and which have an economical profit as compared with antibodies and which can be stored for a long period of time.

It is another object of the present invention to provide an inhibiting compound which can strongly bind to proteins of the SARS coronavirus to inhibit the activity of the SARS coronavirus.

It is a further object of the present invention to provide an agent for detecting and inhibiting SARS coronavirus, comprising the compound as an active ingredient.

It is still a further object of the present invention to provide a method for detecting the SARS coronavirus, comprising the use of the capturing compound.

It is still another object of the present invention to provide a biosensor for diagnosing SARS infection, which comprises the capturing compound as an active ingredient.

In accordance with an aspect thereof, the present invention provides a compound for capturing and inhibiting SARS coronavirus, represented by the following Chemical Formula 1 : 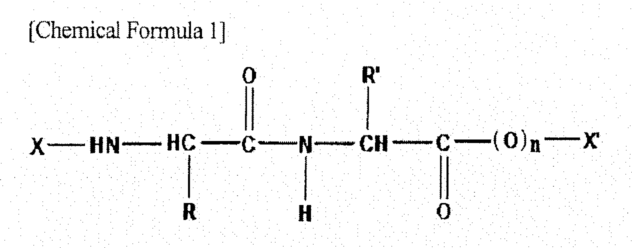 wherein
X and X' are independently H; a functional group selected from a group consisting of biotin, streptavidin and avidin; or a functional moiety composed of the functional group and a linker which links the functional group to the backbone of the compound of Chemical Formula 1 therethrough;
n is 0 or 1; and
R and R' are independently selected from a group consisting of -H, -CH₃, -CH(CH₃)₂, - CH₂CH(CH₃)₂, -CHCH₃CH₂CH₃, -CH₂OH, -CHOHCH₃, -CH₂SH, -(CH₂)₂SCH₃, -CH₂COOH, -CH₂CONH₂, -(CH₂)₂COOH, -(CH₂)₂CONH₂, -(CH₂)₃CH₂NH₂, -(CH₂)₃NHCNHNH₂, 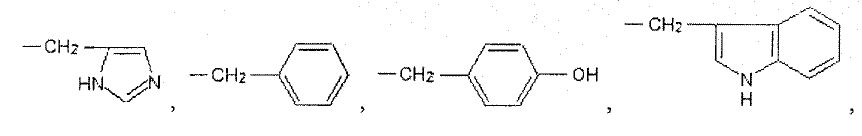 CH₂CH₂CH₂-(proline) and -CH₂SSCH₂-(cystine).

In accordance with another aspect thereof, the present invention provides an agent for detecting and inhibiting SARS coronavirus, comprising the compound of Chemical Formula 1.

In accordance with a further aspect thereof, the present invention provides a method for detecting SARS coronavirus, comprising bringing the compound of Chemical Formula 1 into contact with a sample to be tested.

In accordance with still a further aspect thereof, the present invention provides a biosensor comprising the compound of Chemical Formula 1 as a capturer of the SARS coronavirus.

### BRIEF DESCRIPTION OF THE DRAWINGS

The above and other objects, features and advantages of the present invention will be more clearly understood from the following detailed description made in conjunction with the accompanying drawings, in which:

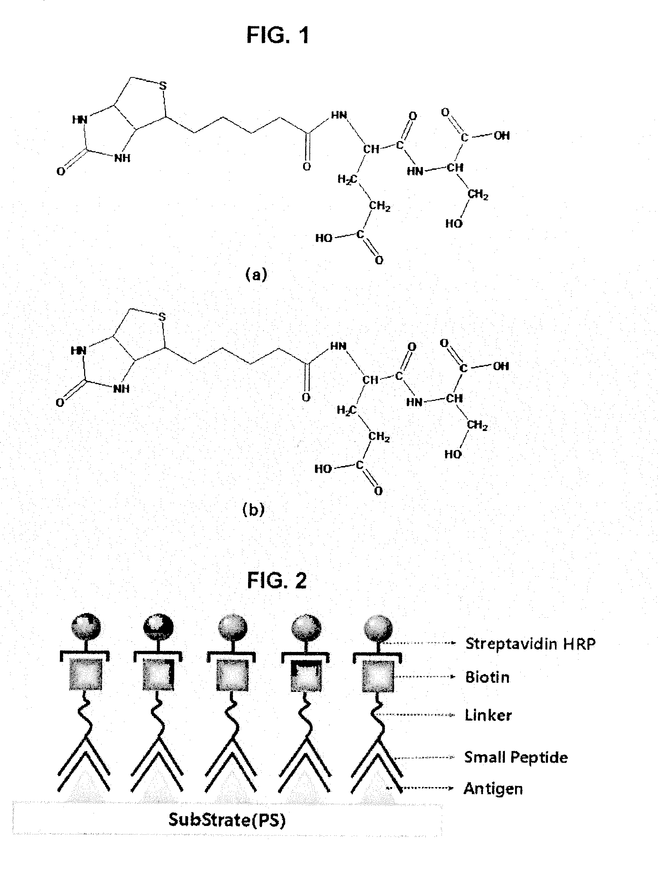

FIG. 1 is a view showing structural formulas of compounds for capturing and inhibiting SARS coronavirus, based on Glu-Ser (SM1) (a) and Glu-Asp (SM2) (b), in accordance with the present invention;
FIG. 2 is a schematic diagram showing a biosensor employing a compound for capturing and inhibiting SARS coronavirus in accordance with the present invention;
FIG. 3 is a schematic diagram showing in a stepwise manner the preparation of a biosensor with a compound for capturing and inhibiting SARS coronavirus in accordance with the present invention;
FIG. 4 is a schematic diagram showing a streptavidin-coated bead for use in detecting SARS coronavirus, modified with a compound for capturing and inhibiting SARS coronavirus in accordance with the present invention;
FIG. 5 is a photograph showing reaction results between compounds, based on 30 amino acid dimers, for capturing and inhibiting SARS coronavirus and two SARS coronavirus protein antigens (ProSci, U.S.A., Catalog Nos. 3533 and 3529).
FIG. 6 is a photograph showing binding forces of the compounds for capturing and inhibiting SARS coronavirus with regard to E-type antigen, a spiked solution of E-type antigen and serum, and human serum, compared to commercial antibodies [SM1: capturing compounds based on Glu-Ser, SM2: capturing compound based on Glu-Asp]; and
FIG. 7 is a graph depicting the results of FIG. 6.

### DESCRIPTION OF THE PREFERRED EMBODIMENTS

The present invention addresses novel compounds for capturing and inhibiting SARS coronavirus, represented by the following Chemical Formula 1 : 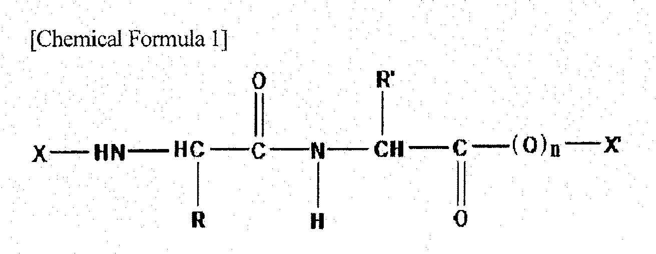 wherein
X and X' are independently H; a functional group selected from a group consisting of biotin, streptavidin and avidin; or a functional moiety composed of the functional group and a linker which links the functional group to the backbone of the compound of Chemical Formula 1 therethrough;
n is 0 or 1; and
R and R' are independently selected from a group consisting or -H, -CH₃, -CH(CH₃)₂, - CH₂CH(CH₃)₂, -CHCH₃CH₂CH₃, -CH₂OH, -CHOHCH₃, -CH₂SH, -(CH₂)₂SCH₃, -CH₂COOH,

-CH₂CONH₂, -(CH₂)₂COOH, -(CH₂)₂CONH₂, -(CH₂)₃CH₂NH₂, -(CH₂)₃NHCNHNH₂, 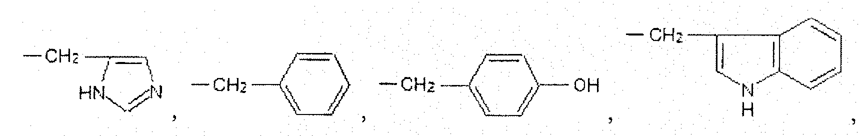 CH₂CH₂CH₂-(proline) and -CH₂SSCH₂-(cystine).

Examples of the linkers useful in the present invention include polyethylene glycols (PEGs), DNA, alkylene of C₁~C₂₀, 1-ethyl-3-(3-dimethylaminopropyl)-carbodiimide (EDC), carbonyldiimidazole (CDI), Sulfo-NhS (Sulfosuccinimidyl), isocyanate derivatives, acylazide derivatives, N-hydroxysuccinimide (NHS), sulfonyl chloride derivatives, aldehyde derivatives, epoxy derivatives, etc.

In greater detail, the linker suitable for X' may include 1-ethyl-3-(3-dimethylaminopropyl)-carbodiimide(EDC), carbonyldiimidazole(CDI), and Sulfo-NHS(Sulfosuccinimidyl), while isocyanate derivatives capable of forming isothiourea bonds, acylazide derivatives capable of amide bonds, N-hydroxysuccininide(NHS), sulfonyl chloride derivatives capable of forming sulfonamide bonds, or aldehyde derivatives or epoxy derivatives capable of forming secondary amide bonds may be used as a linker for X.

The compound of Chemical Formula 1 may be structurally changed by modifying the amine group of the liker or the peptide with an arylating agent, an imidoester, EDC, an anhydride, a fluorophenyl ester, etc.

Preferably, the compound for capturing and inhibiting SARS coronavirus, represented by Chemical Formula 1, may be based on a dipeptide compound selected from a group consisting of Phe-Tyr, Tyr-Trp, Phe-Phe, Phe-Trp, Phe-Leu, Phe-Arg, Gln-Trp; Trp-Phe, Phe-Ala, Trp-Gln, Ala-Ile, Phe-Met, Cys-Phe, Val-Met, Tyr-Gln, Leu-Val, Arg-Phe, Ile-Met, Val-Ala, Lys-Phe, Ser-Cys, Ser-Asp, Asp-Ser, Ser-Glu, Glu-Ser, Asp-Cys, Asp-Glu, Cys-Glu, Cys-Asp, and Glu-Asp.

Also, the present invention pertains to an agent for detecting and inhibiting SARS coronavirus, comprising the compound of Chemical Formula 1 as an active ingredient.

The inhibitory agent of SARS coronavirus may further comprise a pharmaceutically acceptable carrier. As used herein, the term "pharmaceutically acceptable carrier" is intended to refer to a medium applicable to administration as exemplified by solvents, dispersion media, isotonic solutions, absorption delaying agents, etc. The media suitable for use in the administration of active medicinal ingredients are well known in the art. Auxiliary active compounds may be included in the inhibitor.

Also, the present invention pertains to a method for detecting SARS coronavirus, comprising contacting the compound of Chemical Formula 1 with a sample from a subject.

In the method, the contacting step comprises fixing either the compound of Chemical Formula 1 or the sample on a substrate and bringing the compound of Chemical Formula 1 into contact with the sample when it is fixed on the substrate and *vice versa.*

In this case, the contacting step may further comprise adding a label-conjugated secondary capturing material to the substrate when the sample is brought into contact with the compound of Chemical Formula 1 after the compound of Chemical Formula 1 is fixed on the substrate.

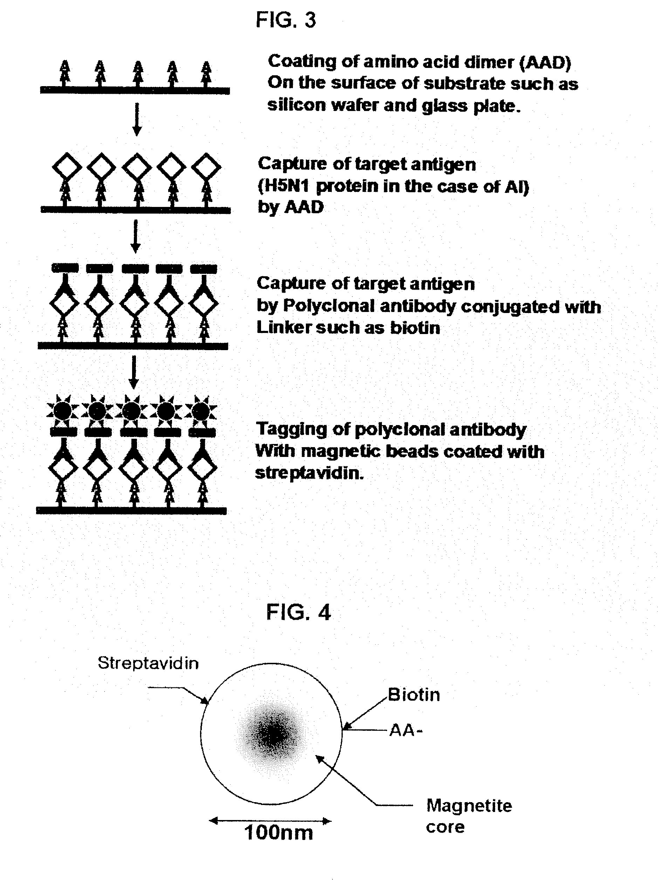

This method is a modification of a sandwich immunoassay as schematically illustrated in FIG. 3.

No limitations are imparted to the substrate so long as it is typically used in the art. Examples include silicon wafer, metal, glass, quartz, etc., but are not limited thereto. In the present invention, the secondary capturing material is intended to mean material such as a polyclonal antibody which can bind to SARS coronavirus captured by the compound of Chemical Formula 1.

In the method, the contacting step may comprise fixing the compound of Chemical Formula 1 on nano- or microparticles; and bringing the sample into contact with the fixed compound of Chemical Formula 1.

In the method, the contacting step may further comprise adding a label-conjugated secondary capturing material to the nano- or microparticles after the sample is brought into contact with the fixed compound of Chemical Formula 1.

So long as it is used in the art, any nano- or microparticle can be used in the present invention. Preferable are magnetic particles.

A better understanding of the present invention may be obtained through the following examples which are set forth to illustrate, but are not to be construed as limiting the present invention.

### EXAMPLE 1: Preparation of Dipeptides

For use in capturing SARS coronavirus proteins or peptides, the compound of the present invention was prepared through the following steps:
Step 1: Structural analysis of SARS coronavirus proteins or peptides to be targeted;
Step 2: Based on the analyzed structure, computational chemistry is used to model a variety of ligands which are expected to bind to the target proteins or peptides and suggest thermodynamically stable compounds upon virtually binding them to the target proteins or peptides;
Step 3: From the suggestions of Step 2, appropriate compounds are selected and synthesized, with the exclusion of compounds which are impossible to practically synthesize or which are already known about;
Step 4: The binding of the synthetic compounds of Step 3 to SARS coronavirus proteins (3533P and 3529P, commercially available from ProSci Inc., U.S.A.) is tested and they are remodeled into forms which are applicable to sensors; and
Step 5: Blinding of the remodeled compounds to the SARS coronavirus proteins (3533P and 3529P, commercially available from ProSci Inc., U.S.A.) is tested to determine whether they can be used as detectors of SARS coronavirus.
In Step 2, binding properties of various compounds virtually synthesized on a computer was confirmed and In Step 3, dipeptides which were practically synthesizable and obtainable at low expense were selected and synthesized.

In the remodeling of Step 4, the linker and the anchor, both used to apply the designed dipeptides to a sensor, may change the binding properties of the designed peptides. The binding properties of the remodeled compounds were therefore analyzed using practical SARS coronavirus antigens in Step 5.

However, because WHO strongly recommends Biosafety Level 3 (BSL3) as the appropriate containment level for working with live SARS-CoV material, antigenic materials available from a world-wide authorized company, such as those identified as ProSci 3533P and 3529P provided by ProSci Inc. U.S.A., were used to test the binding properties of the capturing compounds.

In the binding test of Step 5, it is very important to find the remodeled compounds which show such selectivity that they react with target antigens, but do not bind to the other proteins present in the body. In this example, hence, binding properties of the remolded compounds were tested not only to target antigens but also to the human sera obtained from the Medical School of Eulji University and to spiked solutions prepared by mixing the antigens and the human sera, and the results were compared to the binding properties of SARS coronavirus antibodies.

### 1. Selection of Amino Acid dimers

1) Reference was made to the RCSB protein data bank data (PDB ID NO 2AMQ) concerning the structures of target proteins (antigens), and the sequences of practically used antigens were ordered from ProSci, U.S.A.
2) Water molecules were removed from the obtained proteins using an ADT program version 4.0 (Autodock Tool, http://autodock.scripps.edu) and the water-free target proteins were subjected to virtual association with all of the known 441 amino acid dimers.
3) Excellent virtual binding properties made selection of 30 among the 441 amino acid dimers and they were ordered with 95% or higher purity from SeouLin Bioscience Inc., Korea (synthesized in Thermo Science).

### 2. Preparation of Capturing Compounds

1) Selected amino acid dimers were diluted to 1.0 mM in PBS.
2) Each of the amino acid dimer solutions (1.0 mM) were mixed with one volume of a 1.0 mM NHS-sulfo-biotin solution (PBS) to prepare a 0.5 mM amino acid dimer solutions.
3) The 0.5 mM amino acid dimer solutions were incubated at 30°C for 30 min in an Eppendorf shaking reactor.
4) Again, the 0.5 mM solutions were 50-fold diluted, that is, to 10 µM, which was determined in consideration of the fact that the antibodies provided from ProSCI were about 6.7 µM.
5) The solutions thus prepared were stored in a refrigerator until use without ion removal.

Hereinafter, "capturing compounds" is a term used to refer to amino acid dimers conjugated with a linker + anchor complex (the linker + anchor complex means Sulfo-NHS-Biotin provided from Pierce U.S.A.).

### 3. Coating of antigen

1) The microplates used in this experiment were NUNC Maxisorp microplates. Microplates were used in their original, new state without being washed,
2) For use, the antigens were 1/1000 diluted in a coating buffer (e.g., 10 µL of the antigen solution was mixed with 10 mL of coating buffer).
3) Human sera were 1/10 diluted in a coating buffer.
4) As for the spiked solution (expressed usually as SP on microplates), it was prepared by mixing 50 µL of an antigen stock with 950 µL of human serum. For practical use, it was 1/10 0 diluted in coating buffer.

### 4. Binding Test with Antigen

1) The prepared dilutions of the antigen, the human serum, and the spiked solution each were seeded in an amount of 100 µL per well on microplates and incubated overnight at 20°C.
   Typically, each well of antigen-coated plates was washed twice or three times with autoclaved, deionized water and incubated for 2 hrs at 20°C with 200 µL of blocking buffer. However, when coated with the spiked solution and the human serum, the microplates were washed in the same manner but incubated with PBS, not with blocking buffer because the spiked solution and human serum were used as negative reference for determining the selective binding.
2) The blocking buffer used in this binding test comprised 0.1% BSA and 0.02% thimersol in PBS, pH 7.2 and was not autoclaved. A blocking step, if conducted, was followed by washing three or more times with washing buffer and then twice or more times with PBS.
3) After completion of the washing, the plates were turned upside down and tapped three times against blotting paper so that no residue was left on the plates.
4) Thereafter, the capturing compounds and the antibody solutions each were allocated in an amount of 100 µL, according to the purposes thereof.
5) They were incubated at room temperature for 1 hr with shaking at 100 rpm.
6) Washing was conducted three to five times with a washing buffer and then three to four times with PBS.
7) Step 4) was repeated.
8) Streptavidin-HRP (Horse radish peroxides) and IgG-HRP were 1/10000 diluted in PBS and plated in an amount of 100 µL in each well containing the capturing compound and the antibody.
9) The washing of Step 6) was repeated.
10) The procedure of Step 4) was repeated.
11) 50 µL of a mixture of 1:1 of TMB : substrate solution was allocated.
12) Incubation was conducted at 37°C for 15 min.
13) A stop solution (2N HCl) was added within 10 min after which absorbance was read at 450 nm using a microplate reader.

### 5. Results

**TABLE 1**

| No. | Dimer | M-Type Ranking | M (mean value) | E-Type Ranking | E Value |
|---|---|---|---|---|---|
| 1 | Phe-Tyr | 15 | 0.078 | 26 | 0.127 |
| 2 | Tyr-Trp | 26 | 0.063 | 30 | 0.090 |
| 3 | Phe-Phe | 28 | 0.059 | 29 | 0.116 |
| 4 | Phe-Trp | 22 | 0.071 | 14 | 0.159 |
| 5 | Phe-Leu | 27 | 0.061 | 6 | 0.198 |
| 6 | Phe-Arg | 13 | 0.080 | 11 | 0.167 |
| 7 | Gln-Trp | 8 | 0.093 | 12 | 0.166 |
| 8 | Trp-Phe | 23 | 0.069 | 20 | 0.148 |
| 9 | Phe-Ala | 15 | 0.078 | 10 | 0.175 |
| 10 | Trp-Gln | 19 | 0.073 | 15 | 0.155 |
| 11 | Ala-lle | 30 | 0.057 | 17 | 0.154 |
| 12 | Phe-Met | 29 | 0.059 | 8 | 0.190 |
| 13 | Cys-Phe | 24 | 0.069 | 13 | 0.165 |
| 14 | Val-Met | 7 | 0.094 | 18 | 0.151 |
| 15 | Tyr-Gln | 25 | 0.069 | 27 | 0.122 |
| 16 | Leu-Val | 10 | 0.088 | 21 | 0.147 |
| 17 | Arg-Phe | 21 | 0.072 | 9 | 0.183 |
| 18 | Ile-Met | 18 | 0.074 | 22 | 0.139 |
| 19 | Val-Ala | 17 | 0.077 | 28 | 0.118 |
| 20 | Lys-Phe | 14 | 0.080 | 16 | 0.154 |
| 21 | Ser-Cys | 12 | 0.087 | 19 | 0.149 |
| 22 | Ser-Asp | 9 | 0.091 | 23 | 0.136 |
| 23 | Asp-Ser | 11 | 0.087 | 25 | 0.132 |
| 24 | Ser-Glu | 20 | 0.073 | 24 | 0.135 |
| 25 | Glu-Ser | 6 | 0.111 | 6 | 0.198 |
| 26 | Asp-Cys | 5 | 0.114 | 5 | 0.238 |
| 27 | Asp-Glu | 2 | 0.169 | 2 | 0.520 |
| 28 | Cys-Glu | 4 | 0.143 | 3 | 0.356 |
| 29 | Cys-Asp | 3 | 0.144 | 4 | 0.326 |
| 30 | Glu-Asp | 1 | 0.186 | 1 | 0.552 |

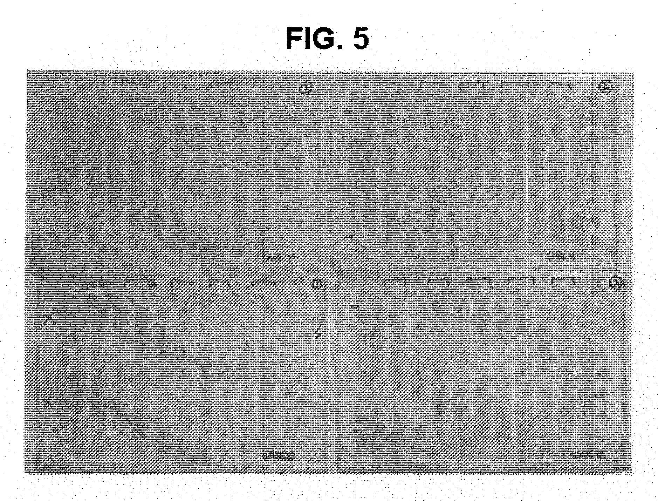

As is apparent from the data of Table 1, the capturing compounds prepared from the 30 selected amino acid dimers are different in binding properties from each other and between the two commercially available antigens (FIG. 5), indicating that the peptides, although very short in length, show different binding properties with regard to antigens.

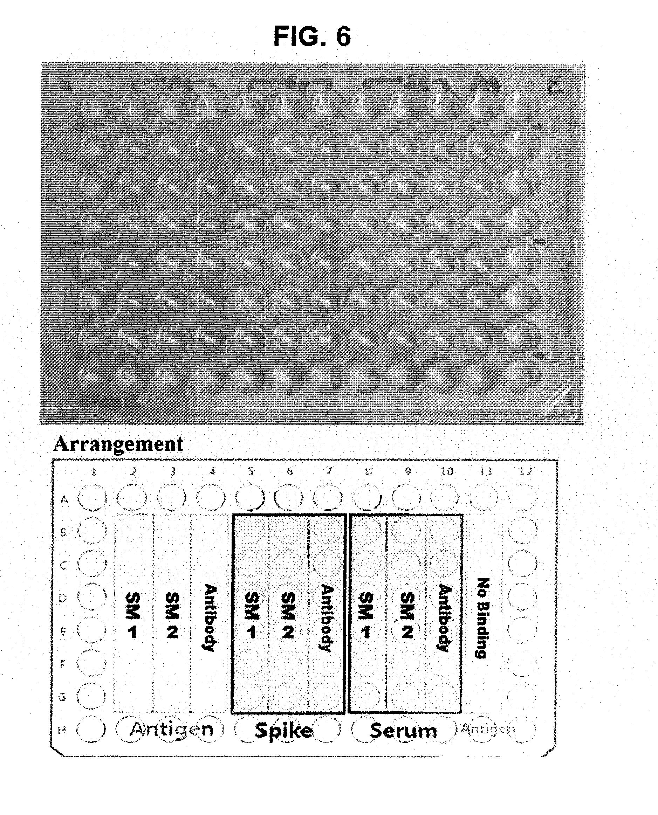

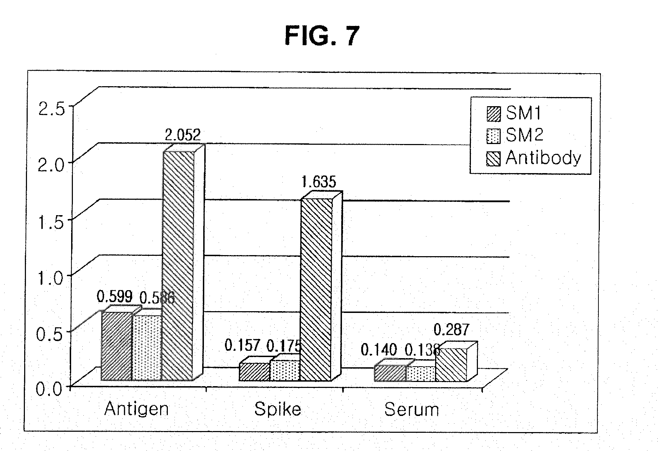

Compared to the antibody, the amino acid dimers of the present invention were inferior in reactivity with the antigens, but superior in selective reactivity because they were far inferior in reactivity with a spiked solution and human serum (FIGS. 6 and 7). Thus, the capturing compounds of the present invention are unlikely to bind to proteins other than the antigens in vivo.

Having an ability to strongly bind to surface compounds of SARS coronavirus, as described above, the peptide compounds in accordance with the present invention show excellent activity of detecting and inhibiting SARS coronavirus in addition to being provided at low cost thanks to the ease of their synthesis. Also, the peptide compounds are so stable that they are not destroyed even after one year of storage. The compounds of the present invention thus overcome the problems generated by the imnune analysis using an antibody, such as low activity or degradation of antibodies, difficulty in analysis at poorly equipped labs, the sacrifice of a variety or numerous animals for producing antibodies, etc. In contrast to antibodies, further, the peptide compounds for detecting and inhibiting SARS coronavirus in accordance with the present invention can be readily applied to biosensors.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. An agent for capturing and inhibiting SARS coronavirus, comprising a compound represented by the following Chemical Formula 1: 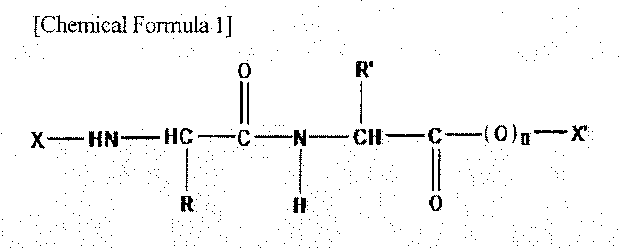 wherein
X and X' are independently H; a functional group selected from a group consisting of biotin, streptavidin and avidin; or a functional moiety composed of the functional group and a linker which links the functional group to the backbone of the compound of Chemical Formula 1 therethrough;
n is 0 or 1; and
R and R' are independently selected from a group consisting of -H, -CH₃, -CH(CH₃)₂, - CH₂CH(CH₃)₂, -CHCH₃CH₂CH₃, -CH₂OH, -CHOHCH₃, -CH₂SH, -(CH₂)₂SCH₃, -CH₂COOH, -CH₂CONH₂, -(CH₂)₂CCOH, -(CH₂)₂CONH₂, -(CH₂)₃CH₂NH₂, -(CH₂)₃NHCNHNH₂, 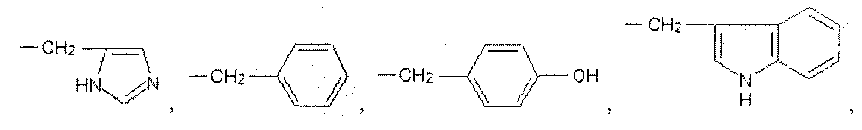 CH₂CH₂CH₂- and -CH₂SSCH₂-,

2. The agent as set forth in claim 1, wherein the linker is selected from a group consisting of polyethylene glycols (PEGs), DNA, alkylenes of C₁-C₂₀, 1-ethyl-3-(3-dimethylaminopropyl)-carbodiimide (EDC), carbonyldiimidazole (CDI); Sulfo-NHS (Sulfosuccinimidyl), isocyanate derivatives, acylazide derivatives, N-hydroxysuccinimide (NHS), sulfonyl chloride derivatives, aldehyde derivatives, and epoxy derivatives.

3. A compound for capturing and inhibiting SARS coronavirus, represented by the following Chemical Formula: 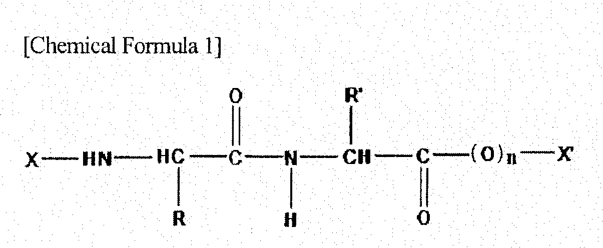 wherein
X and X' are independently H; a functional group selected from a group consisting of biotin, streptavidin and avidin; or a functional moiety composed of the functional group and a linker which links the functional group to the backbone of the compound of Chemical Formula 1 therethrough;
n is 0 or 1; and
R and R' are independently selected from a group consisting of -H, -CH₃, -CH(CH₃)₂, - CH₂CH(CH₃)₂,-CHCH₃CH₂CH₃, -CH₂OH, -CHOHCH₃, -CH₂SH, -(CH₂)₂SCH₃, -CH₂COOH, -CH₂CONH₂, -(CH₂)₂COOH, -(CH₂)₂CONH₂, -(CH₂)₃CH₂NH₂, -(CH₂)₃NHCNHNH₂, 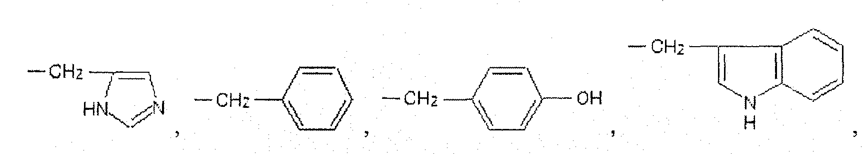 CH₂CH₂CH₂- and -CH₂SSCH₂-.

4. The compound as set forth in claim 3, wherein X and X' are independently a functional group selected from a group consisting of biotin, streptavidin and avidin; or a functional moiety composed of the functional group and a linker which links the functional group to the backbone of the compound of Chemical Formula 1 therethrough:

5. The compound as set forth in claim 3 or 4, wherein the linker is selected from a group consisting of polyethylene glycols (PEGs), DNA, alkylenes of C₁~C₂₀, 1-ethyl-3-(3-dimethylaminopropyl)-carbodiimide (EDC), carbonyldiimidazole (CDI), Sulfo-NHS (Sulfosuccinimidyl), isocyanate derivatives, acylazide derivatives, N-hydroxysuccinimide (NHS), sulfonyl chloride derivatives, aldehyde derivatives, and epoxy derivatives.

6. A method for detecting SARS coronavirus, comprising: contacting a compound, represented by the following Chemical Formula 1, with a sample from a subject: 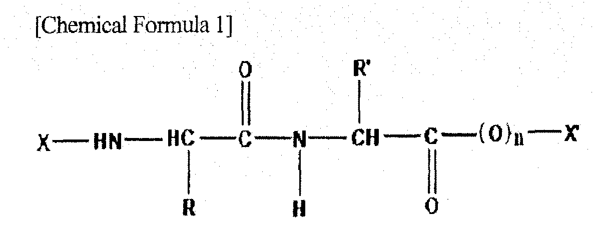 wherein
X and X' are independently H; a functional group selected from a group consisting of biotin, streptavidin and avidin; or a functional moiety composed of the functional group and a linker which links the functional group to the backbone of the compound of Chemical Formula 1 therethrough;
n is 0 or 1; and
R and R' are independently selected from a group consisting of -H, -CH₃, -CH(CH₃)₂, - CH₂CH(CH₃)₂,-CHCH₃CH₂CH₃, -CH₂OH, -CHOHCH₃, -CH₂SH, -(CH₂)₂SCH₃, -CH₂COOH, -CH₂CONH₂, -(CH₂)₂-COOH, -(CH₂)₂CONH₂, -(CH₂)₃CH₂NH₂, -(CH₂)₃NHCNHNH₂, 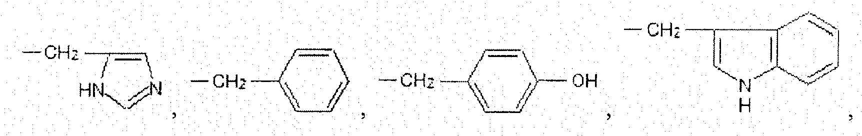 CH₂CH₂CH₂- and -CH₂SSCH₂-,

7. The method as set forth in claim 6, wherein the contacting step comprises:
fixing either the compound of Chemical Formula 1 or the sample on a substrate; and
bringing the compound of Chemical Formula 1 into contact with the sample when it is fixed on the substrate and *vice versa.*

8. The method as set forth in claim 7, wherein the contacting step further comprises adding a label-conjugated secondary capturing material to the substrate when the sample is brought into contact with the compound of Chemical Formula 1 after the compound of Chemical Formula 1 is fixed on the substrate.

9. The method as set forth in claim 6, wherein the contacting step comprises:
fixing the compound of Chemical Formula 1 on nano- or microparticles; and
bringing the sample into contact with the fixed compound of Chemical Formula 1.

10. The method as set forth in claim 9, wherein the contacting step further comprises adding a label-conjugated secondary capturing material to the nano- or microparticles after the sample is brought into contact with the fixed compound of Chemical Formula 1.

11. The method as set forth in claim 9 or 10, wherein the nano- or microparticles are magnetic particles.

12. A biosensor for diagnosing SARS infection, comprising the compound of claim 3 as a detector of SARS coronavirus.
